# EUROPÄISCHE PATENTANMELDUNG

(11) **EP 2 020 235 A1**
(43) Veröffentlichungstag der Anmeldung: **04.02.2009**
(21) Anmeldenummer: 08012748.3
(22) Anmeldetag: 15.07.2008
(51) Int. Cl.: A61K 31/569, A61P 9/00, A61P 9/12, A61K 9/16, A61K 9/20

(54) **einphasisches pharmazeutisches kombinationspräparat (dienogest und ethinylestradiol) zur oralen therapie der regulierung des blutdruckes**

(30) Priorität: 31.07.2007 US 952940 P; 01.08.2007 DE 102007036516
(71) Anmelder: Bayer Schering Pharma AG, 13353 Berlin (DE)
(72) Erfinder: Zimmermann, Thomas, 07747 Jena (DE); Mittmann, Katrin, 07745 Jena (DE)
(74) Vertreter: Cramer, Eva-Maria

(57) **Zusammenfassung**

Einphasische orale Kontrazeptiva, enthaltend eine Kombination von 2.0 mg Dienogest vom Typ eines Nicht-Aldosteron-Antagonisten und 0.030 mg Ethinylestradiol oder 2.0 mg Dienogest und 0.020 mg Ethinylestradiol oder 2.0 mg Dienogest und 0.015 mg Ethinylestradiol oder 1.5 mg Dienogest und 0.015 mg Ethinylestradiol zu mindestens 21 Tagesdosiseinheiten realisieren eine Regulierung des Blutdruckes - Senkung eines erhöhten Blutdruckes, Erhöhung eines niedrigen Blutdruckes - während der Kontrazeption.

Sie sind besonders zur kontrazeptiven Langzeitanwendung ohne Risiko der negativen Beeinflussung des Blutdrucks geeignet.

## Beschreibung

### Technisches Gebiet

Die Erfindung betrifft ein Verfahren zur Herstellung eines einphasischen pharmazeutischen Präparates zur oralen Therapie der Regulierung des Blutdruckes, wobei eine kontrazeptive Kombination von 2.0 mg 17α-Cyanomethyl-17-β-hydroxyestra-4,9-dien-3on (Dienogest) und 0.030 mg 17α-Ethinylestradiol (Ethinylestradiol) oder 2.0 mg Dienogest und 0.020 mg Ethinylestradiol oder 2.0 mg Dienogest und 0.015 mg Ethinylestradiol oder 1.5 mg Dienogest und 0.015 mg Ethinylestradiol zu mindestens 21 Tagesdosiseinheiten verwendet wird.

### Stand der Technik

Es ist bekannt, dass kardiovaskuläre Erkrankungen ein teilweise unterschätztes Gesundheitsproblem sind. Beispielsweise halten 35 % potentieller Patientinnen in den USA den Brustkrebs und nur 7 % kardiovaskuläre Erkrankungen für ihr größtes Gesundheitsrisiko.

Einer der entscheidenden Risikofaktoren der kardiovaskulären Erkrankung ist die Hypertonie. Jedoch ist auch die Hypotonie nicht außer Acht zu lassen, da diese in ihren Symptomen, wie Müdigkeit, Antriebslosigkeit, Schwäche, Schwindel und Ohnmachtsneigung erheblichen Einfluss auf die Lebensqualität der Betroffenen ausüben kann.

Ist der Blutdruck zu niedrig, kann es zu einer ungenügenden Blut- und damit Sauerstoffversorgung von Herz, Gehirn und anderen Organen kommen. Für Schwangere bedeutet niedriger Blutdruck ein Risikofaktor, denn es besteht ursächlich ein Zusammenhang zwischen erniedrigten Blutdruck, ungenügender Uterusdurchblutung sowie Entwicklungsstörungen und perinatalen Komplikationen.

Selbstverständlich soll sowohl die Hypertonie als auch die Hypotonie primär vom Kardiologen mit entsprechenden Mitteln behandelt werden. Aber auch Gynäkologen werden zukünftig noch intensiver den Zusammenhang zwischen Hypertonie/Hypotonie und Kontrazeption, Hormonsubstitution oder die Behandlung gynäkologischer Erkrankungen betrachten.

In nachfolgender Tabelle 1 sind die nach European Society of Hypertension, 2003 - Kategorisierten Blutdruckwerte aufgeführt.

| Kategorie | systolisch (mmHg) | diastolisch (mmHg) |
|---|---|---|
| optimale Werte | < 120 | < 80 |
| Normale Werte | 120 - 129 | 80 - 84 |
| Hohe Normalwerte | 130 -139 | 85 - 89 |
| Grad 1 Hypertonie (mild) | 140 - 159 | 90 - 99 |
| Grad 2 Hypertonie (moderat) | 160 - 170 | 100 - 109 |
| Grad 3 Hypertonie (schwer) | ≥ 180 | ≥ 110 |
| Isolierte systolische Hypertonie | ≥ 140 | 90 |

Hypotonie (niedriger Blutdruck) - zunächst nur ein Messwert und keine Erkrankung - wird von der Weltgesundheitsorganisation WHO bei Frauen bei einem Blutdruck von kleiner 100/60 mg Hg definiert.

Es ist bekannt, dass Estrogene, besonders Ethinylestradiol, das Renin-Angiotensin-Aldosteron-System (RAAS), welches den Blutdruck reguliert, durch vermehrte Bildung von Renin-Substrat (Angiotensinogen) aktivieren. Sie können damit eine Natriumretention unterstützen und eine Blutdruckerhöhung bewirken (Oelkers, W.: Drospirenon: ein neues Gestagen...., Geburtshilfe und Frauenheilkunde 2001; 61: 851-856).

Auch Leidenberger, F. et al (Hrsg):Klinische Endokrinologie für Frauenärzte, 3. vollst. u. erw. Aufl..Springer Medizin Verlag Heidelberg, 2005, 192 erklärt, dass Ethinylestradiol enthaltende orale Kontrazeptiva Wirkung auf das Renin-Angiotensin-System besitzen und deshalb eine Blutdruckerhöhung bewirken können.

Ketelhut, R.G. et al: "Langfristiges Absetzen oraler Kontrazeptiva ..., J. Hypertonie 2000; 4(2), 18-21, weist darauf hin, dass orale Kontrazeptiva den Blutdruck erhöhen, wobei nach Beendigung der Gabe dieser Kontrazeptiva eine Senkung des Blutdrucks zu verzeichnen ist.

Mueck A.O. et al: "Hormontherapie und Hypertonie", J Hypertonie 2006; 10(1), 14-21 erklärt ebenfalls, dass orale Kontrazeptiva, besonders bei Langzeiteinnahme, häufig einen Anstieg des Blutdrucks, mehr des systolischen als des diastolischen bewirken können. Dabei kann die Entwicklung des Bluthochdrucks sehr rasch nach Einnahme des oralen Kontrazeptivums erfolgen, jedoch kann der Blutdruckanstieg sukzessive und langsam verlaufen.

Elger, W. et al beschreibt in DE 30 22 337, dass orale Kontrazeptiva sich negativ auf den Blutdruck auswirken, dass jedoch die Spironolactonverbindung Drospirenonp (ursprünglich ein Diuretikum vom Typ des Aldosteron-Antagonisten) einen Blutdruckanstieg vermeidet.

Auch Oelkers (s.o.) zeigt auf, dass antimineralkortikoid wirksame Gestagene, wie beispielsweise die Spironolactonverbindung Drospirenon aldosteronantagonistisch und damit natriuretisch und eher blutdrucksenkend wirken.

### Darstellung der Erfindung

Der Erfindung liegt die Aufgabe zugrunde, ein geeignetes Mittel mit kontrazeptiver Wirksamkeit ohne negativen Einfluss auf den Blutdruck bereit zu stellen.

Diese Aufgabe wird erfindungsgemäß durch ein Verfahren zur Herstellung eines einphasischen pharmazeutischen Präparates nach Anspruch 1 zur oralen Therapie der Regulierung des Blutdruckes gelöst., welches eine kontrazeptive Kombination von 2.0 mg Dienogest und 0.030 mg Ethinylestradiol oder 2.0 mg Dienogest und 0.020 mg Ethinylestradiol
oder 2.0 mg Dienogest und 0.015 mg Ethinylestradiol oder 1.5 mg Dienogest und 0.015 mg Ethinylestradiol zu mindestens 21 Tagesdosiseinheiten enthält.

Vorteilhafte Ausgestaltungen der Erfindung bestehen in den Merkmalen der Patentansprüche 2 und 3.

Die orale Arzneiform kann eine Tablette, eine Tablette mit einem Filmüberzug (Filmtablette) oder eine Tablette mit einer zuckerhaltigen Hülle (Dragee) sein.

Auch sind unter die erfindungsgemäßen peroralen Arzneiformen zu zählen: Hartgelatinekapsel, Weichgelatinekapsel mit öligen oder wässrigen Suspensionen als Füllmasse oder andere perorale Suspensionen.

Die Freisetzung der Wirkstoffe, bzw. das Herauslösen dieser aus der Tablettenmarix/ Tablettenkern wird mit dem Dissolutionstest unter Verwendung von Wasser mit 37 °C als Dissolutionmedium und 50 U/Min als Rührgeschwindigkeit bestimmt.

Die Bestimmung erfolgt nach Ph.Eur. mittels Blattrührerapparatur unter Verwendung von 1000 ml Wasser.
Ferner wird die Aufgabe durch ein Kit nach Anspruch 4 gelöst.

Das Kit nach Anspruch 4 kann außerdem zusätzlich 7 oder weniger einnahmefreie oder placebohaltige Tagesdosiseinheiten enthalten. Diese sind zur Gabe im Anschluss an die Dauer von mindestens 21 aufeinander folgenden Tagen bestimmt, so dass die Gesamtzahl an Tagesdosiseinheiten 28 beträgt.

Auch kann nach Anspruch 5 die Anzahl der Tagesdosiseinheiten mit der Kombination von Dienogest und Ethinylestradiol 21, 22, 23, 24, oder 25 und die einnahmefreie oder placebohaltige Tagesdosiseinheiten 7, 6, 5, 4 oder 3 betragen.

Die Anzahl der Tagesdosiseinheiten kann nach Anspruch 6 und 7 mindestens nx21 tägliche Dosiseinheiten der kontrazeptiven Kombination nach Anspruch 1 gemeinsam mit einem oder mehreren pharmazeutisch annehmbaren Hilfsstoffen/Trägern mit n gleich 2, 3, 4, 5, 6 ,7, 8, 9, 10, 11, 12, 13, 14, 15, 16, und 17 und maximal 7 , aber auch 3, 4, 5 oder 6 tägliche einnahmefreie oder placebohaltige Dosiseinheiten betragen.

Abhängig vom Wunsch der Frau nach kontinuierlicher Regulierung des Blutdrucks und einer kontinuierlichen Kontrazeption verbunden mit dem Bedürfnis nach Blutungsfreiheit über einen größeren Zeitraum kann nach Anspruch 8 die Anzahl der Tagesdosiseinheiten, mit der Kombination von Dienogest und Ethinylestradiol 84 betragen, wobei die einnahmefreien oder placebohaltigen Tagesdosiseinheiten 7 betragen, so dass die Gesamtzahl an Zyklustagen pro Jahr 4x( nx21 plus 7) mit n gleich 4 ist.

Es wurden überraschenderweise und erfindungsgemäß blutdruckregulierende (Senkung eines erhöhten Blutdruckes, Erhöhung eines niedrigen Blutdruckes) orale Kontrazeptiva gefunden, welche Ethinylestradiol und Dienogest vom Typ eines Nicht-Aldosteron-Antagonisten enthalten. Diese pharmazeutischen Kombinationen sind besonders zur Langzeitanwendung ohne Risiko der negativen Beeinflussung des Blutdrucks geeignet.
Mit der erfindungsgemäßen Verwendung der pharmazeutischen Kombination aufgezeigt in Anspruch 1 zur Herstellung einer pharmazeutischen Zusammensetzung zur Blutdruckregulierung wird auch für Frauen, welche eine Kontrazeption wünschen und die unter leichtem Bluthochdruck leiden bzw. bei denen die Einnahme oraler Kontrazeptiva zur Erhöhung des Blutdrucks führt ein geeignetes Mittel ohne negative Beeinflussung ihres Blutdruckes bereit gestellt.

Ferner wird für Frauen mit erhöhtem bzw. niedrigem Blutdruck, welche vorerst eine Schwangerschaft wünschen und nachfolgend einen Kinderwunsch realisieren möchten mit der Verwendung der pharmazeutischen Kombination nach Anspruch 1 eine Möglichkeit der Verminderung des Risikos von Entwicklungsstörungen Ungeborener (Föten) und perinatalen Komplikationen bei Eintritt einer Gravidität eröffnet.

Für ebendiese Frauen ist gleichfalls durch Zugabe von Metafolin zur pharmazeutischen Kombination mit der Verwendung der Kombination auch eine Möglichkeit der Verminderung des Risikos folatmangelbedingter angeborener Fehlbildungen bei Eintritt einer Gravidität gegeben, wie beispielsweise wie Neuralrohrdefekten, Lippen-Kiefer-Gaumenspalten, und Schwangerschaftskomplikationen, wie Plazentaablösung und Frühgeburtlichkeit..

### Ausführungsbeispiele zur pharmazeutischen Kombination

### Beispiel 1

Valette ist ein konventionelles Dragee zur oralen Kontrazeption, enthaltend 0.030 mg Ethinylestradiol und 2.0 mg Dienogest in einem Tablettenkern, der mit einer zuckerhaltigen Hülle überzogen ist.

### Beispiel 2

2 mg Dienogest und 0.02 mg Ethinylestradiol, wobei 1 mg Dienogest verzögert und 1 mg Dienogest und 0.02 mg Ethinylestradiol schnell freigesetzt werden.

Das Beispiel beschreibt eine Filmtablette mit Matrixkern. Der Kern der Filmtablette enthält 1 mg Dienogest in einer hydrophilen Erosionsmatrix mit dem Grundbestandteil Metolose. Die Matrix gibt den Wirkstoff Dienogest retardiert frei. Der Kern wurde mit einem schnell löslichen Film überzogen, der 1.0 mg Dienogest und 0.02 mg Ethinylestradiol enthält. Zum Lichtschutz wurde die Filmtablette mit einer weiteren schnell löslichen, Eisenoxidpigmente enthaltenden Farbschicht überzogen.

### Zusammensetzung:

| Kern | | Filmhülle | |
|---|---|---|---|
| Granulat 1 | | Film 1 - wirkstoffhaltig | |
| Dienogest | 1.000 mg | Dienogest | 1.000 mg |
| Metolose 90 SH-4000 | 7.500 mg | Ethinylestra diol | 0.020 mg |
| Laktose-Monohydrat | 21.000 mg | Methocel 5 | 2.250 mg |
| Maisstärke | 14.000 mg | Talkum | 0.450 mg |
| Povidon K25 (10% in Ethanol) | 1.500 mg | Titandioxid | 0.280 mg |
| Granulat 2 | | Film 2 - Farbschicht | |
| Laktose-Monohydrat | 54.000 mg | Methocel 5 | 3.375 mg |
| Maisstärke | 27.100 mg | Talkum | 0.675 mg |
| Maltodextrin (25% in Wasser) | 6.900 mg | Titandioxid | 1.875 mg |
| äußere Phase | | Eisenoxid, rot | 0.075 mg |
| Carboxymethylstärke-Natrium | 1.500 mg | | |
| Magnesiumstearat | 1.500 mg | | |

### Beispiel 3

Die Filmtablette der Gesamtdosis 1.5 mg und 0.015 mg Ethinylestradiol besteht aus einem retardierenden Matrixkern und einer schnell löslichen Filmhülle sowie einer Farbschicht.

### Zusammensetzung:

| Kern | 104 mg |
|---|---|
| Dienogest | 0.675 mg |
| Metolose 90SH-4000 | 9.000 mg |
| Laktose-Monohydrat | 42.925 mg |
| Maisstärke | 15.000 mg |
| Povidon K25 (19% in Wasser) | |
| Maltodextrin (25% in Wasser) | 6.000 mg |
| Tablettose | 8.500 mg |
| Avicel PH 102 | 7.000 mg |
| Magnesiumstearat | 0.900 mg |

| Snerrschicht | - |
|---|---|
| Eudragit RL 30 D (als Lacktorcken-Substanz) | |
| Macrogol 6000 | |
| Talkum | |

| wirkstoffhaltiger Film | |
|---|---|
| Dienogest | 0.825 mg |
| Ethinylestradiol | 0.015 mg |
| Methocel 5 | 4.060 mg |
| Talkum | 0.836 mg |
| Titandioxid | 0.264 mg |

| Farbschicht | |
|---|---|
| Methocel 5 | 1.500 mg |
| PEG=Macrogol 6000 | 0.300 mg |
| Talkum | 0.300 mg |
| Titandioxid | 0.850 mg |
| Eisenoxid, rot | 0.050 mg |

### Beispiel 4

Es werden Tabletten mit folgender Zusammensetzung herstellt:

| Kern: | | |
|---|---|---|
| Dienogest | 2.000 mg | oder 1.500 mg |
| Ethinylestradiol | 0.015 mg | |
| Metafolin | 0.451 mg | |
| Laktose-Monohydrat | 28.720 mg | |
| Maisstärke | 15.000 mg | |
| Maltodextrin | 3.750 mg | |
| Magnesiumstearat | 0.500 mg | |

Als Ethinylestradiol kann auch ein Ethinylestradiol-beta-Cyclodextrin-Komplex eingesetzt werden. Im Falle der Verwendung des Ethinylestradiolbeta-Cyclodextrin-Komplex (1:2) ist maximal bzw. in etwa die zehnfache Menge einzusetzen.

Alle Substanzen werden in geeigneter Weise gemischt und granuliert. Es erfolgt das Aufziehen des Metafolin nach Abschluss des Granulationsprozesses, erneutes Mischen, Tablettierung und gegebenenfalls Befilmung.

### Beispiel 5

Es werden Tabletten mit folgender Zusammensetzung herstellt:

| | |
|---|---|
| Kern: | |
| Dienogest | 2.000 mg |
| Ethinylestradiol | 0.030 mg |
| Metafolin | 0.451 mg |
| Laktose-Monohydrat | 28.720 mg |
| Maisstärke | 15.000 mg |
| Maltodextrin | 3.750 mg |
| Magnesiumstearat | 0.500 mg |

Als Ethinylestradiol kann auch ein Ethinylestradiol-beta-Cyclodextrin-Komplex eingesetzt werden. Im Falle der Verwendung des Ethinylestradiolbeta-Cyclodextrin-Komplex (1:2) ist maximal bzw. in etwa die zehnfache Menge einzusetzen.

Alle Substanzen werden in geeigneter Weise gemischt und granuliert. Es erfolgt das Aufziehen des Metafolin nach Abschluss des Granulationsprozesses, erneutes Mischen, Tablettierung und gegebenenfalls Befilmung.

### Untersuchungen zur Wirksamkeit der beanspruchten Formulierungen Darstellung der Abbildungen

Die Erfindung wird unter Bezugnahme auf die beigefügten Abbildungen genauer beschrieben.

In Abb. 1 ist der mittlere systolische Blutdruck in Abhängigkeit von der Behandlung und der Visite der Behandlungen A und B aufgezeigt, wobei S gleich Screening; B gleich Baseline; 8 gleich Woche 8; 12 gleich Woche 12; 25 gleich Woche 25; 38 gleich Woche 38; F gleich Abschlussvisite bedeutet.

In Abb. 2 ist der mittlere diastolische Blutdruck in Abhängigkeit von der Behandlung und der Visite der Behandlungen A und B aufgezeigt, wobei S gleich Screening; B gleich Baseline; 8 gleich Woche 8; 12 gleich Woche 12; 25 gleich Woche 25; 38 gleich Woche 38; F gleich Abschlussvisite bedeutet.

Es ist zu beachten, dass bei den Untersuchungen zur Wirksamkeit der beanspruchten Formulierungen hauptsächlichst Augenmerk auf die Gruppen ,Hohe Normalwerte' bis ,milde Hypertonie-Grad1/"borderline"' nach der Tabelle 1 gelegt wurde, da Probandinnen der Hypertonie-Gruppen ,Stadium2/3' bzw. ,Grad 2/3' aus Sicherheitsgründen in Wirksamkeitsstudien für Kontrazeptiva nicht eingeschlossen bzw. davon ausgeschlossen wurden.

Tabelle 2 definiert explizit die Untergruppen für den systolischen Blutdruck

**Tab 2**

| Systolischer Blutdruck bei Baseline [mmHg] | Systolischer Blutdruck bei Screening [mmHg] | Untergruppe |
|---|---|---|
| < 110 | < 110 | niedrig |
| < 110 | 110 - 129 | niedrig/normal |
| 110 - 129 | < 110 | niedrig/normal |
| 110 - 129 | 110 - 129 | normal |
| ≥ 130 | 110 - 129 | hoch/normal |
| 110 - 129 | ≥ 130 | hoch/normal |
| ≥ 130 | ≥ 130 | hoch |
| < 110 | ≥ 130 | niedrig/hoch |
| ≥ 130 | <110 | niedrig/hoch |

In einer randomisierten, doppelt-blinden klinischen Studie wurden 1315 Frauen im Alter zwischen 18 und 41 Jahren , die ihr schriftliches Einverständnis zur Studienteilnahme gegeben hatten, mit 2 unterschiedlichen Behandlungen, behandelt.

Die Behandlung (A) entsprach der Kombination - 2 mg Dienogest/ 30 µg Ethinylestradiol - mit einer kontinuierlichen Einnahme von täglich 1 Tablette über einen Langzyklus zu 84 Tagen gefolgt von 7 Tagen Pause zu insgesamt 4 Langzyklen.

Die zweite Behandlung (B) entsprach der Kombination - 2 mg Dienogest/30 µg Ethinylestradiol - mit einer kontinuierlichen Einnahme von täglich 1 Tablette über einen Zyklus zu 21 Tagen gefolgt von 7 Tagen Pause (konventionelle Einnahme) zu insgesamt 13 konventionellen Zyklen.

Die Auswertung der Blutdruckwerte erhoben jeweils bei Screening, bei Baseline, in der 6.-8. Behandlungswoche, in der 10.-12. Behandlungswoche, in der 23.-25. Behandlungswoche, in der 36.-38. Behandlungswoche und bei der Abschlussvisite (im Regelfall nach einem Jahr Behandlung) ergab folgendes:

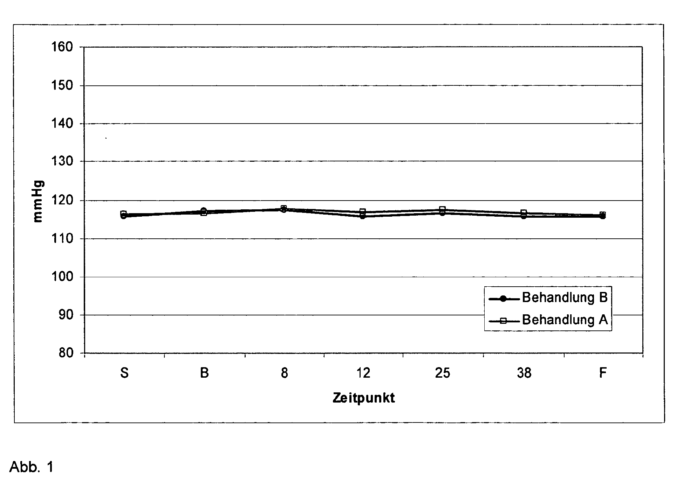

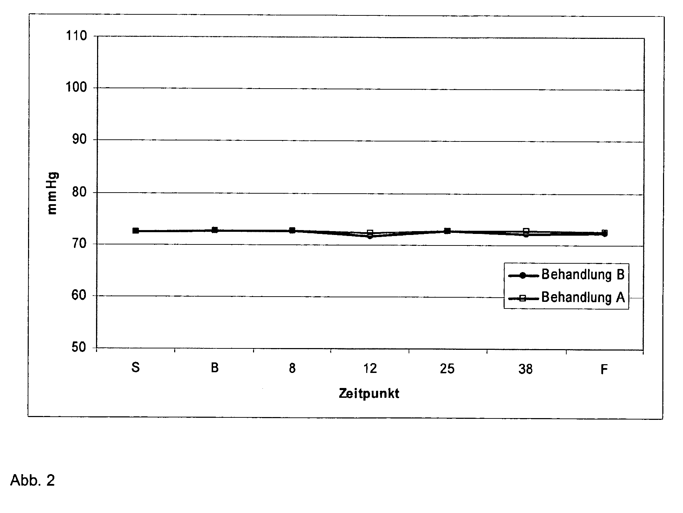

Der mittlere systolische Blutdruck und der mittlere diastolische Blutdruck blieben sowohl in Gruppe A als auch in Gruppe B im gesamten Studienverlauf nahezu konstant (siehe Abb. 1 und 2).

Betrachtet man die Entwicklung des mittleren systolischen Blutdrucks in den gemäß Tabelle 2 gebildeten Untergruppen hinsichtlich der Blutdruckwerte bei Baseline und Screening (siehe Abb. 2), so zeigen Frauen mit eingangs niedrignormalen Werten im Mittel einen diskreten Anstieg des systolischen Blutdrucks.

Frauen mit eingangs hochnormalen Werten bzw. leicht erhöhten Werten erfuhren im Mittel einen diskreten Abfall des systolischen Blutdrucks.

Bei Frauen mit Blutdruckwerten im mittleren Normalbereich wurden diese durch Medikation im Mittel nur unwesentlich beeinflusst.
Mit den gemäß Tabelle 2 gebildeten Untergruppen gilt gleiches für den diastolischen Blutdruck.

## Patentansprüche

1. Verfahren zur Herstellung eines einphasischen pharmazeutischen Präparates zur oralen Therapie der Regulierung des Blutdruckes,
**dadurch gekennzeichnet, dass**
eine kontrazeptive Kombination mit 2.0 mg Dienogest und 0.030 mg Ethinylestradiol oder 2.0 mg Dienogest und 0.020 mg Ethinylestradiol oder 2.0 mg Dienogest und 0.015 mg Ethinylestradiol oder 1.5 mg Dienogest und 0.015 mg Ethinylestradiol zu mindestens 21 Tagesdosiseinheiten verwendet wird.

2. Verfahren nach Anspruch 1,
**dadurch gekennzeichnet, dass**
die Arzneiform des einphasischen pharmazeutischen Präparates eine Filmtablette darstellt, bestehend aus einem Tablettenkern mit einem retardierend freizusetzenden anteiligem Gehalt zum Gesamtgehalt an Dienogest und einem Filmüberzug mit einem nicht retardierend (schnell) freizusetzenden anteiligem Gehalt zum Gesamtgehalt an Dienogest und einem nicht retardierend (schnell) freizusetzenden Gesamtgehalt an Ethinylestradiol.

3. Verfahren nach den Ansprüchen 1 bis 2,
**dadurch gekennzeichnet, dass** aus der Arzneiform mindestens 10 %, vorzugsweise 30 % an Dienogest retardiert nach größer 30 Minuten aus dem Tablettenkern heraus gelöst werden, wie mit dem Dissolutionstest unter Verwendung von Wasser mit 37 °C als Dissolutionmedium und 50 U/Min als Rührgeschwindigkeit bestimmt.

4. Kit nach den Ansprüchen 1 bis 3,
enthaltend mindestens 21 tägliche Dosiseinheiten der kontrazeptiven Kombination gemeinsam mit einem oder mehreren pharmazeutisch annehmbaren Hilfsstoffen/Trägern und maximal 7 tägliche einnahmefreie oder placebohaltige Dosiseinheiten.

5. Kit nach den Ansprüchen 1 bis 4,
wobei die Anzahl der Tagesdosiseinheiten mit der Kombination von Dienogest und Ethinylestradiol 21, 22, 23, 24 oder 25 beträgt und wobei die einnahmefreien oder placebohaltigen Tagesdosiseinheiten 7, 6, 5, 4 oder 3 , so dass die Gesamtzahl an Zyklustagen 28 beträgt.

6. Kit nach den Ansprüchen 1 bis 5,
enthaltend mindestens nx21 tägliche Dosiseinheiten der kontrazeptiven Kombination gemeinsam mit einem oder mehreren pharmazeutisch annehmbaren Hilfsstoffen/Trägern mit n gleich 2, 3, 4, 5, 6 ,7, 8, 9, 10, 11, 12, 13, 14, 15, 16, und 17 und maximal 7 tägliche einnahmefreie oder placebohaltige Dosiseinheiten.

7. Kit nach den Ansprüchen 1 bis 6,
wobei die Anzahl der täglichen
einnahmefreien oder placebohaltigen Dosiseinheiten 3, 4, 5, 6 und 7 beträgt.

8. Kit nach den Ansprüchen 1 bis 7,
wobei die Anzahl der Tagesdosiseinheiten mit der Kombination von Dienogest und Ethinylestradiol 84 beträgt und wobei die einnahmefreien oder placebohaltigen Tagesdosiseinheiten 7 betragen, so dass die Gesamtzahl an Zyklustagen pro Jahr 4x( nx21 plus 7) mit n gleich 4 ist.
